# EUROPEAN PATENT SPECIFICATION

(11) **EP 1 684 837 B1**
(45) Date of publication and mention of the grant of the patent: **05.08.2009**
(21) Application number: 04794466.5
(22) Date of filing: 07.10.2004
(51) Int. Cl.: A61M 25/00

(54) **GUIDE CATHETER**
FÜHRUNGSKATHETER
CATHETER-GUIDE

(30) Priority: 09.10.2003 US 682220
(43) Date of publication of application: 02.08.2006
(73) Proprietor: Medtronic, Inc., Minneapolis, MN 55432 (US)
(72) Inventor: SHAH, Sonar, Minneapolis, MN 55403 (US); GOODE, Johnson, E., Maple Grove, MN 55311 (US); KELLEY, James, F., Coon Rapids, MN 55448 (US); SCHNEIDER, Mark, D., Mound, MN 55364 (US); JANNICKE, Jeff, Andover, MN 55304 (US)
(74) Representative: Hughes, Andrea Michelle
(86) International application number: PCT/US2004/033124
(87) International publication number: WO 2005/035043

(56) References cited:
- US-A- 5 289 831
- US-A1- 2003 109 823
- US-A1- 2003 114 732
- US-B1- 6 221 059

## Description

The invention relates generally to catheters and, more particularly, to guide catheters for introducing medical devices and or agents into a body of a patient.

Guide catheters are used to position medical devices, such as catheters and electrode leads, and or to place auxiliary implant tools, such as guide wires, and or to infuse agents such as therapeutic fluids or contrast media in desired locations within the body of a patient. A guide catheter typically includes an elongated sheath defining an inner channel through which the medical devices or agents are delivered, once the sheath has been inserted into the body.

To enable precise positioning of a guide catheter, the guide catheter often includes radio-opaque and or echogenic portions so that, using fluoroscopic or ultrasonic imaging techniques, the physician can visualize the guide catheter. Fully radiopaque or fully radiopaque and echogenic guide catheter distal tips, which may also further facilitate navigation through tortuous anatomy, are particularly desirable. Such a guide catheter is disclosed in US 2003/0109823.

The invention provides a guide catheter as claimed in claim.

### BRIEF DESCRIPTION OF DRAWINGS

The following drawings are of particular embodiments of the invention and therefore do not limit its scope, but are presented to assist in providing a proper understanding of the invention. The drawings are not to scale (unless so stated) and are intended for use in conjunction with the explanations in the following detailed description.
FIG. 1 is a perspective view of a guide catheter.
FIG. 2 is a cross-sectional side view of the guide catheter of FIG. 1 illustrating incorporation of a radio-opaque and echogenic material in the distal tip.
FIG. 3 is an enlarged cross-sectional side view of the distal tip of the guide catheter shown in FIGS. 1 and 2.
FIG. 4 is a side view of the guide catheter of FIG 1 with an exposed illustration of a reinforcing braid with radio-opaque strands.
FIG. 5 is a cross-sectional view of the inner lumen and sheath of the guide catheter of FIG. 1.
FIG. 6A is a plan view of an elongated sheath of a guide catheter according to embodiments of the present invention.
FIG. 6B is an axial section view of a distal portion of the sheath shown in FIG.6A.
FIG. 7 is a plan view of a guide catheter wherein the sheath shown in FIGs. 6A-B is slideably received within an outer sheath according to some embodiments of the present invention.
FIGs. 8A-D are plan views of a distal portion of a guide catheter elongated sheath according to alternate embodiments of the present invention.
FIGs. 9A-B are plan views of a distal portion of a guide catheter elongated sheath according to further alternate embodiments of the present invention.
FIG 10 is a plan view of a distal portion of a guide catheter elongated sheath according to yet another embodiment.
FIG. 11A is a schematic view of a guide catheter positioned within a chamber of a heart according an embodiment of the present invention.
FIG. 11B is a schematic view of guide catheter elongated sheath cannulating a coronary sinus according to another embodiment of the present invention.
FIGs. 11C-E are schematic views of a guide catheter positioned in the coronary vasculature according to additional embodiments of the present invention.

### DETAILED DESCRIPTION

The following detailed description is exemplary in nature and is not intended to limit the scope, applicability, or configuration of the invention in any way. Rather, the following description provides a practical illustration for implementing exemplary embodiments of the invention.

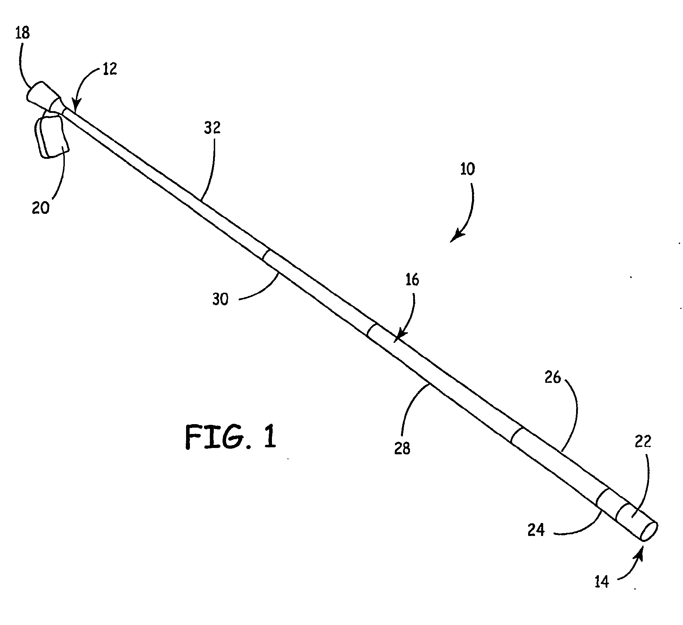

FIG. 1 is a perspective view of a guide catheter 10. As shown in FIG. 1, guide catheter 10 includes a proximal end 12, distal tip 14, and an elongated sheath 16 extending between the proximal and distal ends. Guide catheter 10 is sized for insertion into a lumen, such as a blood vessel, within the human body. Guide catheter 10 defines an inner channel (not shown in FIG. 1) through which other elements such as catheters and electrode leads may be inserted. A luer fitting 18 and handle 20 may be coupled to proximal end 12 of catheter 10. A slitter (not shown) may be positioned near proximal end 12, e.g., adjacent handle 20.

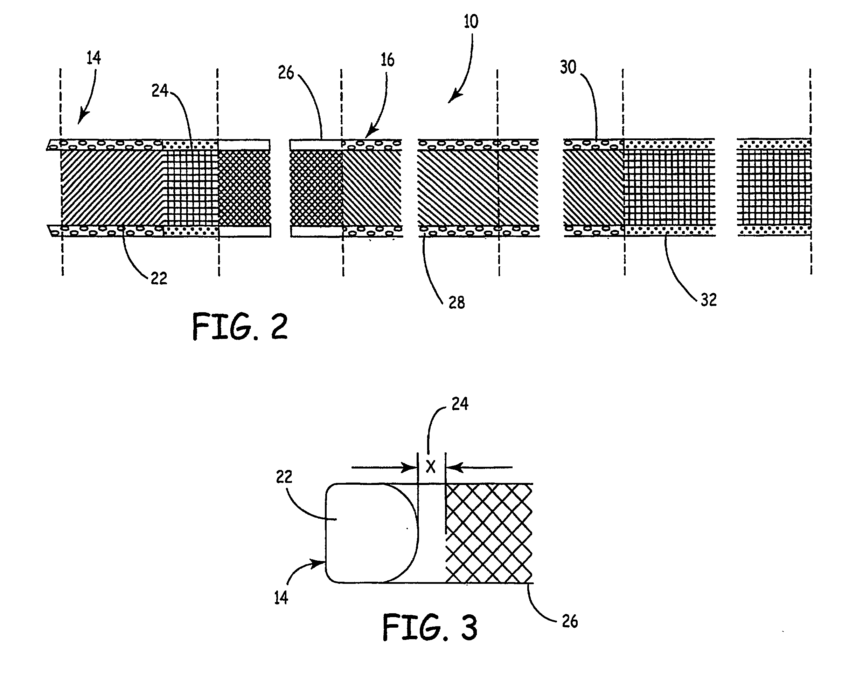

FIG. 2 is a cross-sectional side view of the guide catheter of FIG. 1 illustrating incorporation of a radio-opaque and echogenic material in the distal tip of guide catheter 10. As shown in FIGS. 1 and 2, sheath 16 may include a number of sheath segments 22, 24, 26, 28, 30, 32 disposed along the length of catheter 10. Sheath 16 may be formed to provide either a straight or pre-bent shape to guide catheter 10, depending on the desired end application.

Sheath 16 is made from a material that permits slitting along the length of catheter 10 and promotes maneuverability. In particular, each sheath segment 22, 24, 26, 28, 30, 32 may be constructed of a polymeric material, such as polyether block amide, nylon block polymer, silicone, or polyurethane, as well as composites or mono-polymers. An example of one suitable polymeric material is the polyether block amide marketed under the trademark PEBAX® and commercially available from Atofina Chemicals Inc., of King of Prussia, Pennsylvania.

Guide catheter 10 is constructed to exhibit properties that promote enhanced visibility of the catheter 10 using fluoroscopic or ultrasonic imaging techniques. With reference to FIG. 1, sheath segment 22 forms a distal tip of guide catheter 10, and incorporates a material that is both fluoro and echo visible. The material also may be provided along the length of the guide catheter. For example, the material may be distributed continuously along the length of catheter 10 or at intermittent positions.

The material incorporated in distal tip 22 is tungsten carbide, which exhibits both radio-opacity and echogenicity. For enhanced echogenicity, the tungsten carbide may be jet milled and has an average particle size of less than approximately 500 nanometers and, more preferably, less than approximately 200 nanometers. The particle size may refer generally to a diameter of the tungsten carbide particles, although spherical particles are not necessary and the particle size may refer to a maximum width dimension. Particle sizes in the above ranges provide increased surface area for reflection of ultrasonic energy, thereby enhancing visibility of portions of guide catheter 10 in which the particles are dispersed. At the same time, tungsten carbide is highly radio-opaque, and facilitates fluoroscopic imaging of guide catheter 10.

The same tungsten carbide particles can be incorporated along the length of guide catheter 10 in sheath segments 24, 26, 28, 30, 32. In particular, the tungsten carbide particles can be dispersed in polymeric material that is molded or extruded to form sheath 16. Alternatively, in one embodiment, the tungsten carbide may be provided in sheath segment 22 in distal tip 14 and sheath segment 24, with the remaining sheath segments 26, 28, 30, 32 carrying barium sulfate particles.

Each sheath segment 22, 24, 26, 28, 30, 32 may be constructed from a similar material with a similar concentration of tungsten carbide particles. However, sheath segments 22, 24, 26, 28, 30, 32 may have different hardness characteristics. As a particular illustration, sheath segments 22, 24, 26, 28, 30, 32 may be constructed from PEBAX material with 25, 35, 55, 63 and 72 Shore D hardnesses, respectively. The tungsten carbide particles can be added to sheath segments 22, 24, 26, 28, 30, 32 in a concentration on the order of approximately 40 to 75 percent by weight without significantly degrading the overall mechanical properties of guide catheter 10.

As one particular example, the tungsten carbide may be added to the polymeric material in the amount of approximately 70 to 75 percent by weight and, more preferably, approximately 73 to 74 percent by weight. In an exemplary embodiment, the jet milled tungsten carbide material is added to the polymeric material in a weight of approximately 73.2 percent by weight. A concentration of 73.2 percent by weight tungsten carbide particles to PEBAX™ Shore D material corresponds to a concentration of approximately 15 percent by volume. The barium sulfate particles may be added to sheath segments 26, 28, 30, 32 in the amount of approximately 25 to 35 percent by weight and, more preferably, approximately 30 percent by weight.

The jet milled tungsten carbide particles offer exceptional echogenicity and, when added to the polymeric material, permit ready slitting along the length of guide catheter 10. For these reason, in determining the concentration of tungsten carbide particles, it is desirable to balance the degree of echogenicity against the slittability of sheath 16. As more tungsten carbide particles are added to segments 22, 24, 26, 28, 30, 32, the material forming guide catheter 10 becomes difficult to process and, in some cases, difficult to maneuver for insertion into and removal from the body of a patient. A guide catheter 10 constructed as described herein retains desirable mechanical properties, enabling ease of maneuverability and atraumatic use.

FIG. 3 is an enlarged cross-sectional side view of distal tip 14 of guide catheter 10. As shown in FIG. 3, distal tip 14 may comprise a sheath segment 22 that is separated from sheath segment 26 by in intermediate sheath segment 24. Sheath segment 24 may provide a spacing "x" between sheath segments 22, 26. In particular, a reinforcing braid (not shown in FIG. 3) may extend along substantially the entire length of guide catheter 10 and be embedded between inner and outer walls of sheath 16. However, the distal end of the reinforcing braid terminates in sheath segment 26, and does not extend into distal tip 14. The distance "x" may be on the order of 0.25 to 0.35 inches (0.64 to 0.89 centimeters).

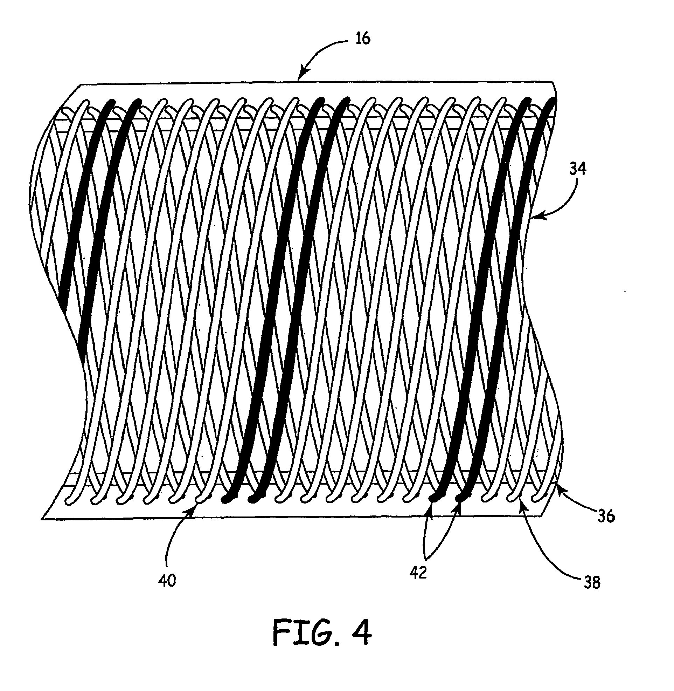

FIG. 4 is a side view of guide catheter 10 with an exposed illustration of a reinforcing braid 34. Reinforcing braid 34 is substantially tubular in shape, and includes an inter-woven array of strands 40. Braid 34 may be formed between inner wall 36 and outer wall 38 of sheath 16. To promote visibility, some of the strands may be radio-opaque. In particular, in the example of FIG 4, two strands 42 are formed from a radio-opaque material. The radio-opaque material use in strands 42 may be formed from a variety of materials such as platinum iridium, gold, tantalum, platinum, tungsten carbide, and the like. Strands 40 may be formed from a variety of conventional metallic materials such as steel, or plastic materials such as polyester. Thus, the radio-opaque material is formed into strands that are braided among the steel strands. Incorporation of a relatively small number of strands, e.g., one to three strands, can significantly improve fluoro visibility of the guide catheter.

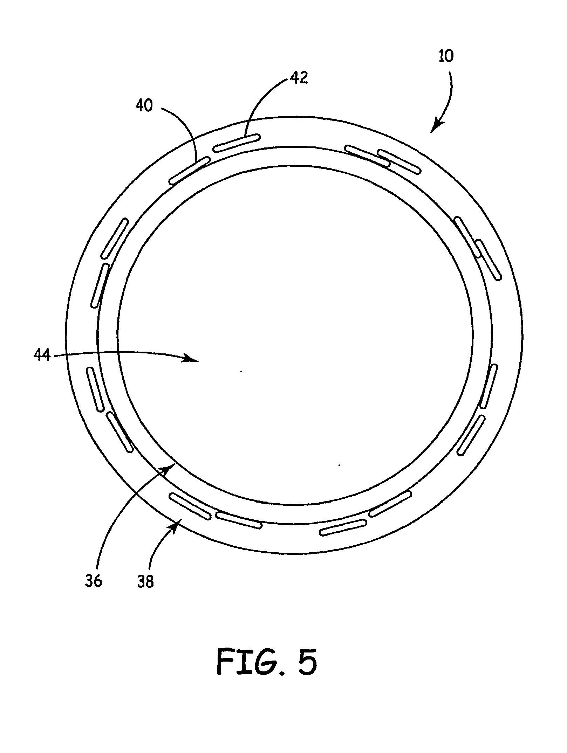

FIG. 5 is a cross-sectional view of the inner channel 44 and sheath 16 of guide catheter 10. Incorporation of strands 42 promotes visibility but does not significantly affect the mechanical properties of guide catheter 10. With strands 42, guide catheter 10 remains readily slittable, unlike other types of guide catheters that may use radio-opaque marker bands. In addition, use of radio-opaque strands 42 offsets the need to provide additional radio-opaque material within walls 36, 38.

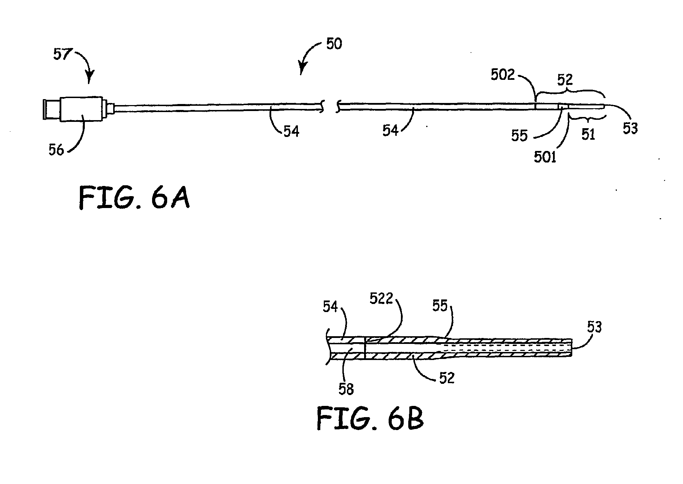

FIG. 6A is a plan view of an elongated sheath 50 of a guide catheter.

FIG. 6A illustrates elongated sheath 50 including a proximal portion 54 to which a hub 56 is joined at a proximal end 57 and a distal tip 51 or 52 extending to a sheath distal end 53 and joined to a distal end 501 or 502 of proximal portion 54, respectively. Materials forming proximal portion 54 of sheath 50 include but are not limited to polyether block amide, nylon block polymer, silicone, or polyurethane. An example of one suitable polymeric material is the polyether block amide marketed under the trademark PEBAX® and commercially available from Atofina Chemicals Inc., of King of Prussia, Pennsylvania. Proximal portion 54 may further incorporate a reinforcing braid, for example braid 34 as described in conjunction with FIGs. 4 and 5.

According to the present invention, distal tip 51 or 52 is fully radiopaque or fully radiopaque and echogenic, having radiopaque or radiopaque and echogenic particles dispersed within a polymer, for example PEBAX®, forming tip. It should be understood that the term "fully" preceding "radiopaque" and "radiopaque and echogenic" is used to describe distal tips according the present invention wherein an entirety of a material forming the distal tips includes either radiopaque particles / filler or radiopaque and echogenic particles / filler. Barium sulfate is an example of an appropriate radiopaque filler, which is well known to those skilled in the ait, and tungsten carbide an example of an appropriate radiopaque and echogenic filler, which is described herein. Although tungsten carbide is preferred as a radiopaque and echogenic filler, alternate embodiments of the present invention include a filler comprising tungsten particles and some of the fillers described in commonly assigned U.S. Patent 5,921,933 of Sarkis et al. Sarkis et al. describe echogenic fillers, some of which also happen to be sufficiently radiopaque to be included within the scope of the present invention, for example titanium dioxide and platinum oxide.

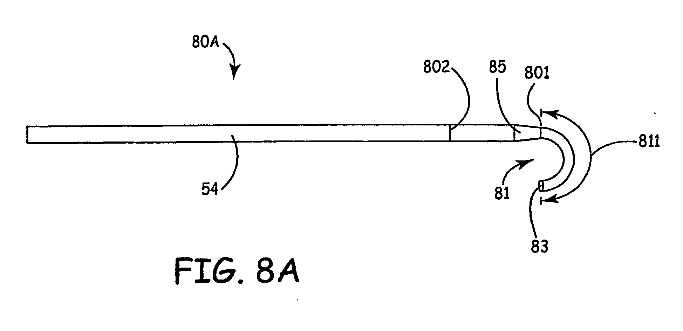

According to another aspect of the present invention, some embodiments include a distal tip, i.e. distal tip 51 or 52, or others described herein below, having a relatively short length, for example between approximately 0.08 inch (2.032 mm) and approximately 0.2 inch (5.08 mm), while, in alternate embodiments, a distal tip is longer than approximately 0.2 inch (5.08 mm), and, in preferred embodiments, having a length between approximately 0.2 inch (5.08 mm) and approximately 2 inches (5.08 cm). Furthermore, according to alternate embodiments, distal tip 51 or 52 includes at least one resilient preformed curve, examples of which are described herein in conjunction with FIGs. 8A-11 E.

According to some embodiments of the present invention, distal tip 51 or 52 is atraumatic to adjacent walls when advanced within structures of a body, that is the tip will tend to give, either compressing or bending, if pushed up against a wall of an internal organ or vessel. Distal tip 51 or 52 may be described as being relatively soft, and, according to one embodiment, has a hardness between approximately 25D and approximately 35D durometer. Furthermore, distal tip 51 or 52 may have a stiffness less than that of proximal portion 54 accomplished by a thinner wall thickness of distal tip 51 or 52, incorporation of a braid reinforcement in proximal portion 54, as previously described, a reduced flexural modulus of material forming distal tip 51 or 52, or any combination thereof. Furthermore, according to some embodiments of the present invention, proximal portion 54 includes at least two stiffnesses wherein a stiffness in proximity to distal end 501 or 502 is less than a stiffness in proximity to proximal end 57.

As is further illustrated in FIG. 6A, sheath 50 includes a tapered transition 55. According to one embodiment, proximal portion 54 includes a tapered transition 55 in proximity to distal end 501 where distal tip, designated as 51, is joined. While, according to an alternate embodiment, distal tip, designated as 52, which is joined to proximal portion 54 at distal end 502, includes tapered transition 55; according to yet another embodiment, tapered transition 55 is not included in either proximal portion 54 or distal tip 52. Tapered transition 55, as well as other tapered transitions described herein, includes only a reduction in outer diameter, according to one embodiment, and both a reduction in inner and outer diameters, according to another embodiment; FIG. 6B illustrates the former and the latter, the latter with dashed lines.

FIG 6B is an axial section view of a distal portion of the sheath shown in FIG. 6A. FIG. 6B illustrates a lumen 58 formed by sheath 50 extending through proximal portion 54 and distal tip 52. Lumen 58 is adapted to slideably engage an implant tool or an electrode lead, a diameter of lumen 58 sized to accommodate one or the other or both, according to alternate embodiments of the present invention; furthermore, lumen 58 may be used to deliver a fluid, either a contrast medium for fluoroscopic visualization or a therapeutic agent.

FIG 6B further illustrates distal tip 52 coupled to proximal portion 54 in a butt joint 522. According to some embodiments of the present invention, tip 52 is heat fused to proximal portion 54 using methods known to those skilled in the art. According to one embodiment, proximal portion 54 and distal tip 52 are placed together on a mandrel, a piece of FEP shrink tubing is placed over butt joint 522, and the assembly is heated to the melting point by a hot air device. Once removed from the heat and cooled, the FEP is stripped off the finished joint. In another embodiment, proximal portion 54 and distal tip 52 are placed on a mandrel in a die, with ends joined as illustrated in FIG. 6B, and radio frequency (RF) energy is applied to heat the die in order to fuse the ends together; the die and or mandrel may also be designed to form taper 55 and or one or more curves in distal tip 52. Although not illustrated, distal tip 52 and proximal portion 54 may be coupled via a lap joint.

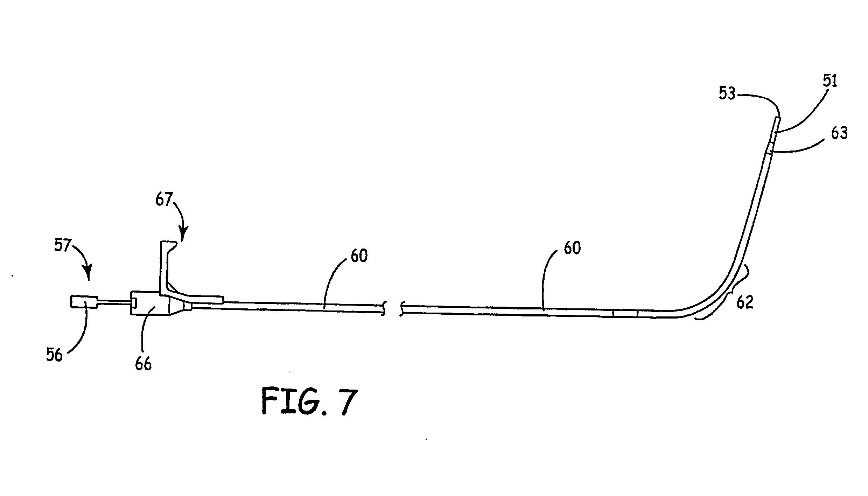

FIG. 7 is a plan view of a guide catheter wherein sheath 50 (FIGs. 6A-B) is slideably received within an outer sheath 60 according to the present invention. FIG. 7 illustrates outer sheath 60 including a proximal end 67, to which a hub 66 is coupled, and a distal end 63; distal tip 51 of sheath 50 protrudes from distal end 63 of sheath 60 having been pushed through outer sheath 60 from proximal end 57, which is shown protruding from proximal end 67 of outer sheath 60. FIG. 7 further illustrates outer sheath 60 including a curve 62 which may either be preformed, that is formed by the manufacturer of the sheath, or formed by a guide catheter operator via a deflection mechanism built into outer sheath 60. Embodiments of outer sheath 60 will typically include braid reinforced polymer walls, similar to that previously described for guide catheter 10 and well known to those skilled in the art. An example of a sheath including a preformed curve is the Medtronic catheter model number 6216A and an example of sheath including a deflection mechanism is the Medtronic catheter model number 6226DEF. According to some embodiments of the present invention, distal end 53 of distal tip 51 of sheath 50 is directed toward a target site via curve 62 of outer sheath 60; a reduced diameter of distal tip 51 may facilitate cannulation of a vessel. Furthermore, if distal tip 51 were to include one or more resilient preformed curves, manipulation, via push and torque, of sheath 50 within outer sheath 60 would further facilitate selective orientation of distal end 53.

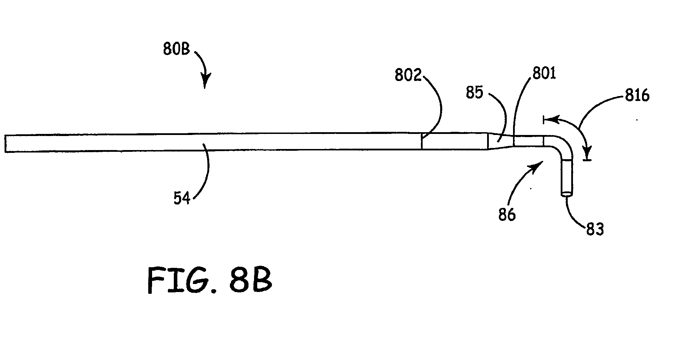

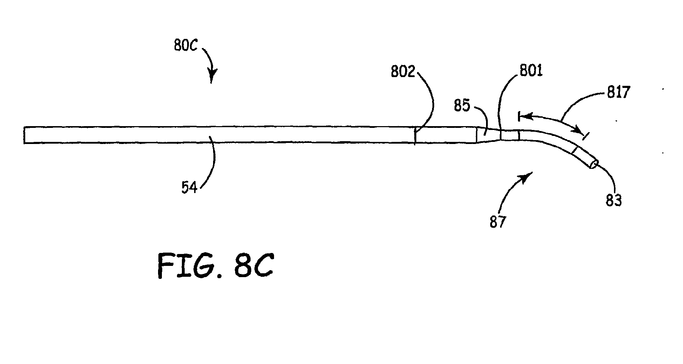

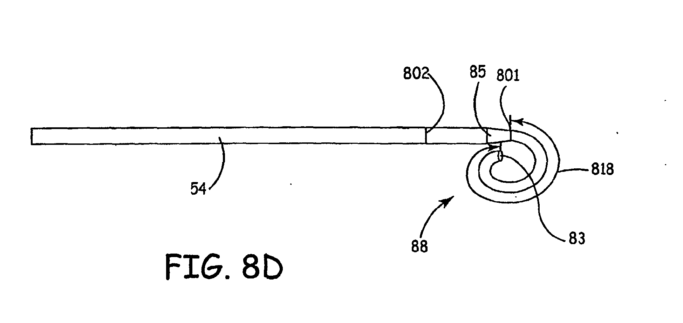

According to alternate embodiments of the present invention, fully radiopaque or fully radiopaque and echogenic distal tips include at least one resilient curve sweeping about an angle from approximately 10° to approximately 360°. It should be noted that angles cited herein are understood to have a tolerance of +/-1 0degrees. FIGs.8A-D are plan views of exemplary distal portions of a guide catheter elongated sheath illustrating some of these embodiments. FIGs. 8A-D illustrate sheaths 80A-D each including proximal portion 54 to which distal tips 81, 8G, 87 and 88, respectively, are coupled via a junction at either a distal end point 801 or a distal end point 802; a tapered transition 85 is present within either proximal portion 54 or within distal tips 81, 86, 87 and 88. According to alternate embodiments, a tapered transition is not included. Each of distal tips 81, 86, 87 and 88 are fully radiopaque or fully radiopaque and echogenic, as previously described in terms of tip 51 or 52, according to alternate embodiments. Furthermore, according to some embodiments tips 81, 86, 87 and 88 are atraumatic as previously described in conjunction with FIG. 6A. FIG. 8A illustrates tip 81 including a resilient preformed curve 811 sweeping about an angle of approximately 180° extending from a point in proximity to point 801 to a distal tip distal end 83. FIG. 8B illustrates tip 86 including a resilient preformed curve 816 sweeping about an angle of approximately 90° from a point in proximity to point 801 to a point in proximity to distal tip distal end 83. FIG. 8C illustrates tip 87 including a resilient preformed curve 817 sweeping about an angle between approximately 10° and approximately 45° from a point in proximity to point 801 to a point in proximity to distal tip distal end 83. FIG. 8D illustrates tip 88 including a resilient preformed curve 818 sweeping about an angle approaching approximately 360° from a point in proximity to point 801 to a point in proximity to distal tip distal end 83.

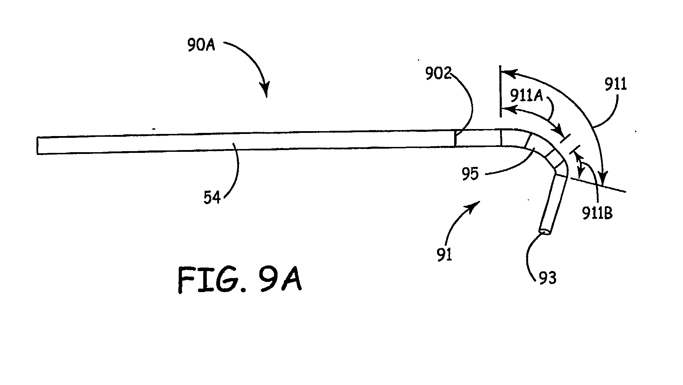

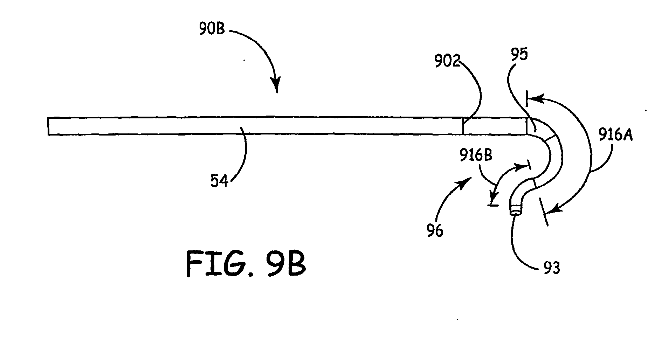

FIGs. 9A-B are plan views of a distal portion of a guide catheter elongated sheath according to further alternate embodiments of the present invention, wherein a distal tip includes two resilient preformed curves. FIG. 9A illustrates sheath 90A including proximal portion 54 to which distal tip 91 is coupled via a junction in proximity to a point 902; distal tip 91 includes a first resilient preformed curve 911A and a second resilient preformed curve 911B forming a compound curve 911, which sweeps about an angle greater than approximately 90° from a point in proximity to point 902 to a point in proximity to a distal tip distal end 93. FIG. 9B illustrates sheath 90B including proximal portion 54 to which distal tip 96 is coupled via a junction in proximity to point 902; distal tip 96 includes a first resilient preformed curve 916A, which sweeps about an angle between approximately 160° and approximately 180°, and second resilient preformed curve 916B, extending in an opposite direction from first curve 916A and sweeping about an angle between approximately 40° and approximately 80°. A combination of curves 916A and 916B in distal tip 96 is alternately described as an Amplatz style curve, which is well known to those skilled in the art. Each of distal tips 91 and 96 are fully radiopaque or fully radiopaque and echogenic, as previously described in conjunction with FIG. 6A, according to alternate embodiments. Furthermore, according to some embodiments tips 91 and 96 are atraumatic as previously described herein.

Although FIGs. 9A-B illustrate distal tips 91 and 96 including a tapered transition 95 located within first curves 911A and 916A, tapered transition 95 may alternately be positioned proximal to first curves 911A and 916A either as part of distal tips 91 and 96 or as a part of proximal portion 54, as previously described herein. Additional embodiments include no tapered transition.

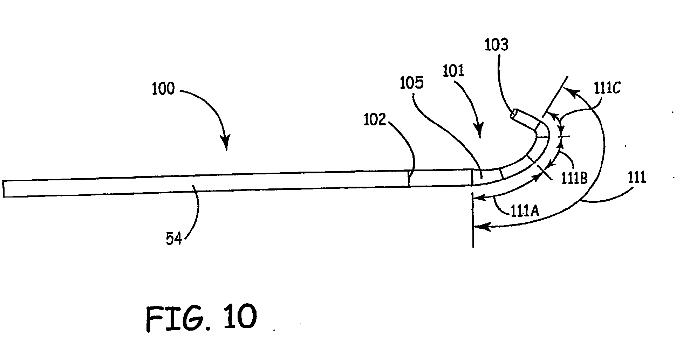

FIG. 10 is a plan view of a distal portion of a guide catheter elongated sheath according to yet another embodiment, wherein a distal tip 101 includes three resilient preformed curves. FIG 10 illustrates sheath 100 including proximal portion 54 to which distal tip 101 is coupled via a junction in proximity to a point 102; distal tip 101 includes a first resilient preformed curve 111A , a second resilient preformed curve 111B and a third resilient preformed curve 111C forming a compound curve 111, which sweeps about an angle greater than approximately 100° from a point in proximity to point 102 to a point in proximity to a distal tip distal end 103. Distal tip 101 is fully radiopaque or fully radiopaque and echogenic, as previously described in conjunction with FIG. 6A, according to alternate embodiments. Furthermore, according to some embodiments tip 101 is atraumatic as previously described herein.

Although FIG 10 illustrates distal tip 101 including a tapered transition 105 located within first curve 111 A, tapered transition 105 may alternately be positioned proximal to first curve 111A either as part of distal tip 101 or as a part of proximal portion 54, as previously described herein. Additional embodiments include no tapered transition.

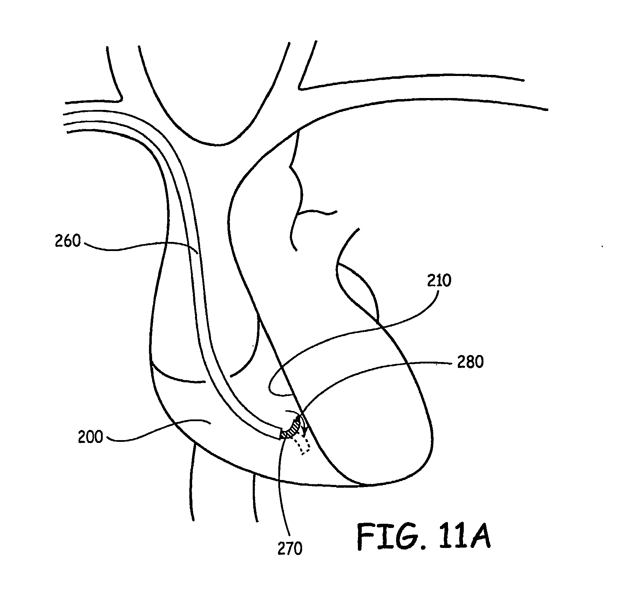

Although elongated sheaths, such as those described in conjunction with FIGs. 6A-10, may be used alone to deliver medical devices or agents, preferred embodiments of guide catheters include such sheaths engaged within an outer sheath, i.e. sheath 60 (FIG 7), in order to facilitate increased maneuverability of distal tips. Furthermore, outer sheaths preferably include a preformed curve or a deflection mechanism, as previously described herein, but the scope of the present invention also includes outer sheaths including no curves, which may engage elongated sheaths having fully radiopaque or radiopaque and echogenic distal tips including resilient preformed curves extending distally from outer sheaths. FIGs. 11A-E illustrate exemplary scenarios in which guide catheters according to the present invention may be employed.

FIG. 11A is a schematic view of a guide catheter positioned within a chamber of a heart according an embodiment of the present invention. FIG. 11A illustrates an outer sheath 260, positioned within a right ventricle 200, from which a distal tip 270 of an elongated sheath, slideably engaged within outer sheath 260, protrudes distally to position a lead 280, slideably engaged therein, for implantation along a septal wall 210. According to embodiments of the present invention, distal tip 270 is fully radiopaque or fully radiopaque and echogenic, and, as illustrated in FIG 11A, includes at least one resilient preformed curve, which is free to reform as distal tip 270 exits outer sheath 260; the preformed curve is conformed to outer sheath 260 as it is advanced therethrough prior to exiting sheath 260. FIG. 11A further illustrates, via dashed lines, that the elongated sheath may be rotated within outer sheath 260 to direct distal tip 270 in an alternate location.

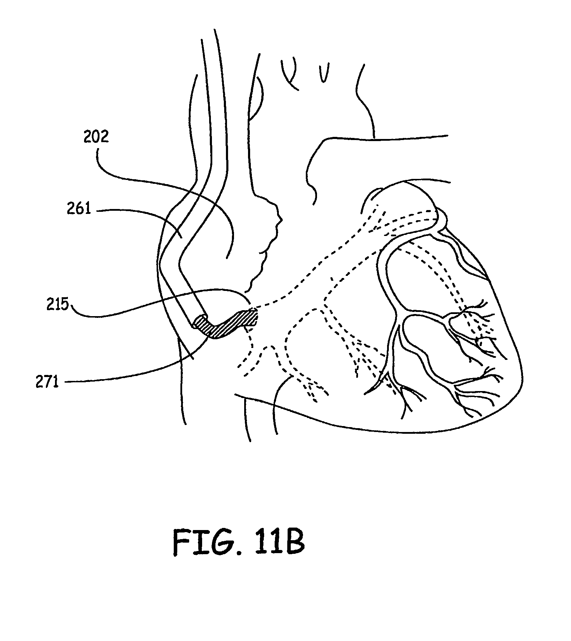

FIG. 11B is a schematic view of guide catheter elongated sheath cannulating a coronary sinus according to another embodiment of the present invention. FIG. 11B illustrates an outer sheath 261, positioned in a right atrium 202, from which a distal tip 271 of an elongated sheath, slideably engaged within outer sheath 261, protrudes distally to cannulate a coronary sinus ostium 215. According to embodiments of the present invention, distal tip 271 is fully radiopaque or fully radiopaque and echogenic, and, as illustrated in FIG. 11B, includes at least two resilient preformed curves, which are free to reform as distal tip 271 exits outer sheath 261; the preformed curves are conformed to outer sheath 261 as they are advanced therethrough prior to exiting sheath 261.

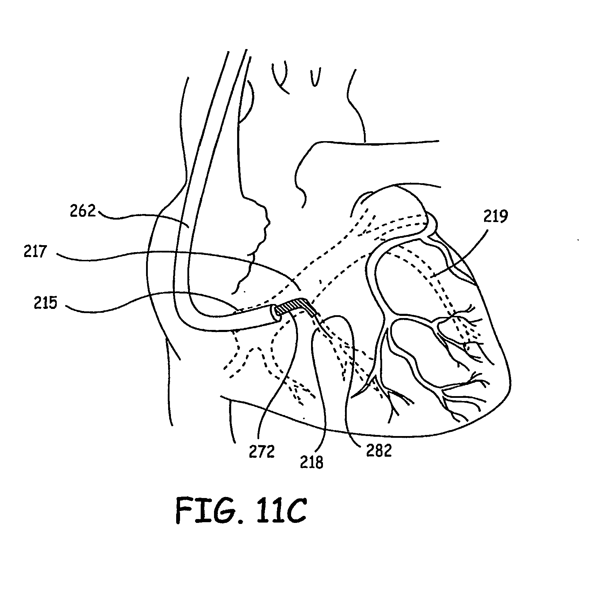

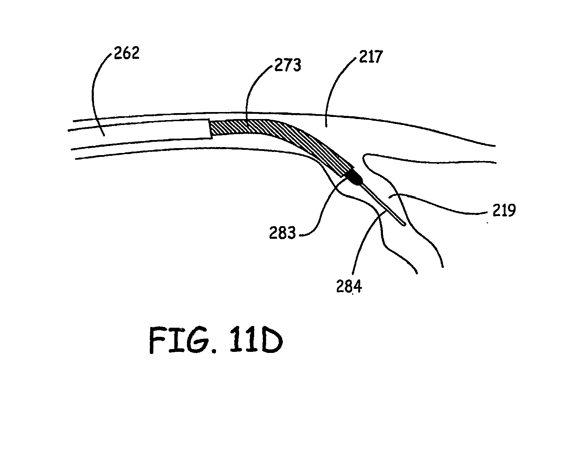

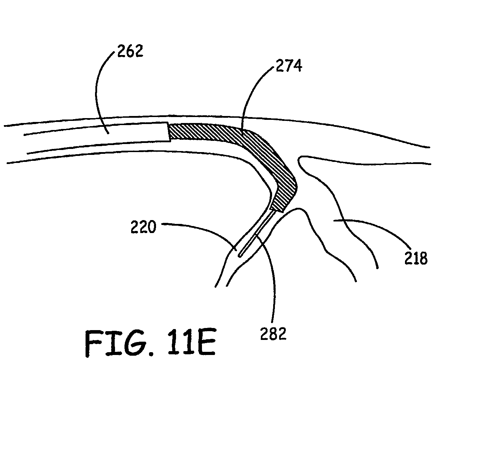

FIGs. 11C-E are schematic views of a guide catheter positioned in the coronary vasculature according to additional embodiments of the present invention. FIGs. 11C-D illustrate an outer sheath 261 having cannulated coronary sinus ostium 215 and advanced within a coronary sinus 217. FIG. 11C a distal tip 272 of an elongated sheath is shown protruding from outer sheath 262 to cannulate a first branch vein 218, while in FIG. 11D a distal tip 273 of a different elongated sheath is shown protruding from outer sheath 262 to cannulate a second branch vein 219 and, in FIG. 11E, a distal tip 274 of yet another elongated sheath is shown protruding from outer sheath 262 to cannulate a secondary branch vein 220. According to embodiments of the present invention, each distal tip 272, 273 and 274 is fully radiopaque or fully radiopaque and echogenic, and, as illustrated in FIGs. 11C-E, include at least one resilient preformed curve, which are free to reform as distal tips 272, 273, and 274 exit outer sheath 262; the preformed curves are conformed to outer sheath 261 as they are advanced therethrough prior to exiting sheath 262. FIGs. 11C further illustrates an elongated element 282 slideably engaged within the sheath including distal tip 272 and extending therefrom into branch vein 218; element 282 is a guide wire or a lead according to alternate embodiments of the present invention. If element 282 is a guidewire, elongated sheath including distal tip 272 may be removed from within outer sheath 262 once the guide wire is in position and a lead passed over the guide wire into branch vein 218. FIG. 11D further illustrates a lead 283 slideably engaged over a guide wire 284 which has been advanced through elongated sheath including distal tip 273 into branch vein 219. Therefore, as previously described in conjunction with FIG. 6B, a lumen of an elongated sheath according to the present invention may be sized to accommodate a guidewire and or a lead. FIG. 11E further illustrates elongated element 282, as previously described in conjunction with FIG. 11C, slideably engaged within the sheath including distal tip 274 and extending therefrom into secondary branch vein 220, which extends from branch vein 218.

According to embodiments illustrated in FIGs. 11B-E distal tips 271, 273, 274 and 274, in addition to directing, by nature of their geometry, may also serve to dilate structures of surrounding coronary vasculature to facilitate cannulation in order that elements, i.e. 282, may be advanced distally therethrough.

### EXAMPLE

A distal tip, for an elongated sheath, having a geometry corresponding to any of the illustrated embodiments is formed by an injection molding process from 3533 SA01 Atofina PEBAX® pellets compounded with 73.2% +/- 2%, by weight, tungsten carbide and 0.25% +/- 0.03%, by weight, Tinuvin 326 UV inhibitor and 0.25% +/- 0.03%, by weight, Irgonox 1010 antioxidant. The tungsten carbide additive is Jet-milled Superfine Tungsten Carbide having an average particle size less than or equal to 200 nanometers (0.2 micron) obtained from Foster Corporation of Dayville, CT. The resulting distal tip has a hardness of approximately 35D.

Although specified at less than or equal to 200 nanometers, tungsten carbide particle sizes in the range of less than or equal to 500 nanometers are within the scope of the present invention providing increased surface area for reflection of ultrasonic energy, thereby enhancing visibility of distal tips. Furthermore, material comprising anywhere from 40% to 80%, by weight, tungsten carbide particles is also within the scope of the present invention. Furthermore, either an extrusion process followed by a secondary forming process or an insert molding process, both methods known to those skilled in the art, may used to form distal tips according to embodiments of the present invention.

In the foregoing detailed description, the invention has been described with reference to specific embodiments. However, it may be appreciated that various modifications and changes can be made without departing from the scope of the invention as set forth in the appended claims. For example, although distal tip curves are illustrated herein in plane with the rest of the sheath, additional embodiments of the present invention include distal tips including curves formed out of plane from the rest of the sheath. Furthermore, the inventors contemplate alternate embodiments of distal tips according to the present invention including an embedded structure, such as a coil.

## Claims

1. A guide catheter comprising an elongated sheath (50) including a fully radiopaque and echogenic distal tip, the distal tip (51) having a length greater than or equal to 0.08 inch (2.032 mm); **characterised in that** said guide catheter further comprising an outer sheath (60) slideably receiving the elongated sheath; wherein the elongated sheath engaged within the outer sheath may be positioned so that the fully radiopaque and echogenic distal tip protrudes from a distal end (63) of the outer sheath.

2. The guide catheter of claim 1, wherein the length of the distal tip is between 0.08 inch (2.032 mm) and 0.2 inch (5.08 mm).

3. The guide catheter of claim 1, wherein the length of the distal tip is greater than or equal to 0.2 inch (5.08 mm).

4. The guide catheter of claim 1, wherein the length of the distal tip is between 0.4 inch (10.16 mm) and 2 inch (5.08 cm).

5. The guide catheter of any preceding claim, wherein the distal tip is formed of a material comprising tungsten carbide particles.

6. The guide catheter of claim 5, wherein the tungsten carbide particles have an average size less than or equal to 500 nanometers.

7. The guide catheter of claim 6, wherein the tungsten carbide particles have an average size between 100 nanometers and 200 nanometers.

8. The guide catheter of claim 6, wherein the tungsten carbide particles have an average size less than or equal to 200 nanometers.

9. The guide catheter of claim 5, wherein the tungsten carbide particles are 40% to 80% by weight of the distal tip.

10. The guide catheter of claim 5, wherein the tungsten carbide particles are 70% to 80% by weight of the distal tip.

11. The guide catheter of claim 1, wherein the elongated sheath further includes a reinforcing braid (34) extending along a length of the sheath to a point proximal to the fully radiopaque and echogenic distal tip.

12. The guide catheter of claim 11, wherein the point proximal to the distal tip is spaced between 0.25 inch (6.35 mm) and 0.35 inch (8.89 mm) from the distal tip.

13. The guide catheter of any preceding claim, wherein the distal tip includes a first resilient preformed curve.

14. The guide catheter of claim 13, wherein the first curve sweeps about an angle greater than or equal to 180°.

15. The guide catheter of claim 13, wherein the first curve sweeps about an angle of 90°.

16. The guide catheter of claim 13, wherein the first curve sweeps about an angle between 10° and 70°.

17. The guide catheter of any of claims 13 to 16, wherein the distal tip further includes a second resilient preformed curve extending from the first resilient preformed curve.

18. The guide catheter of claim 17, wherein the first and second curves, together, sweep about an angle greater than 90°.

19. The guide catheter of claim 17, wherein the first curve sweeps about an angle between 160° and 180° and the second curve sweeps, in an opposite direction to the first curve, about an angle between 40° and 80°.

20. The guide catheter of claim 17, wherein the first curve and the second curve form an Amplatz shape.

21. The guide catheter of any of claims 17 to 20, wherein the distal tip further includes a third resilient preformed curve extending from the second resilient preformed curve.

22. The guide catheter of claim 21, wherein the first, second and third curves, together, sweep about an angle greater than 100°.

23. The guide catheter of any preceding claim, wherein the distal tip includes a tapered transition.

24. The guide catheter of claim 23, wherein the tapered transition is located proximal to the first resilient preformed curve.

25. The guide catheter of claim 23 when dependent on claim 17, wherein the tapered transition is located proximal to the second resilient preformed curve.

26. The guide catheter of claim 23 when dependent on claim 21, wherein the tapered transition is located proximal to the third resilient preformed curve.

27. The guide catheter of any preceding claim, wherein the material forming the distal tip has hardness between 25 durometer and 35 durometer on a D scale.

28. The guide catheter of claim 1, wherein
the elongated sheath further includes a proximal segment; the proximal segment including a distal end; and
the fully radiopaque and echogenic distal tip is coupled to the distal end of the proximal segment by means of a butt joint.

29. The guide catheter of claim 28, wherein the butt joint is heat fused.

30. The guide catheter of claim 29, wherein the butt joint is formed by an RF process.

31. The guide catheter of claim 30, wherein the fully radiopaque and echogenic distal tip includes a tapered transition formed in the RF die.

32. The guide catheter of claim 1, wherein:
the elongated sheath further includes a proximal segment having a first stiffness and including a distal end; and
the fully radiopaque and echogenic distal tip is coupled to the distal end of the proximal segment and has a second stiffness, which is less than the first stiffness.

33. The guide catheter of any preceding claim, wherein the distal tip is atraumatic.

34. The guide catheter of claim 1, wherein
the elongated sheath further includes a proximal segment; the proximal segment including a distal end and a proximal segment outer diameter; and
the fully radiopaque and echogenic distal tip is coupled to the distal end of the proximal segment and includes a distal tip outer diameter, which is less than the proximal segment outer diameter.

## Patentansprüche

1. Führungskatheter mit einer länglichen Hülle (50), die eine vollständig radioopake und echogene distale Spitze beinhaltet, wobei die distale Spitze (51) eine Länge aufweist, die größer oder gleich 0,08 Inch (2,032 mm) ist; **dadurch gekennzeichnet, dass** der Führungskatheter ferner eine äußere Hülle (60) aufweist, die die längliche Hülle gleitend aufnimmt; wobei die längliche Hülle, die mit der äußeren Hülle beaufschlagt ist, so positioniert werden kann, dass die vollständig radioopake und echogene distale Spitze von bzw. aus einem distalen Ende (63) der äußeren Hülle vorsteht.

2. Führungskatheter nach Anspruch 1, bei dem die Länge der distalen Spitze zwischen 0,08 Inch (2,032 mm) und 0,2 Inch (5.08 mm) beträgt.

3. Führungskatheter nach Anspruch 1, bei dem die Länge der distalen Spitze größer oder gleich 0,2 Inch (5,08 mm) ist.

4. Führungskatheter nach Anspruch 1, bei dem die Länge der distalen Spitze zwischen 0,4 Inch (10,16 mm) und 2 Inch (5,08 cm) beträgt.

5. Führungskatheter nach einem der vorstehenden Ansprüche, bei dem die distale Spitze aus einem Material gebildet ist, das Wolframcarbidpartikel aufweist.

6. Führungskatheter nach Anspruch 5, bei dem die Wolframcarbidpartikel eine durchschnittliche Größe von kleiner oder gleich 500 Nanometern aufweisen.

7. Führungskatheter nach Anspruch 6, bei dem die Wolframcarbidpartikel eine durchschnittliche Größe zwischen 100 Nanometern und 200 Nanometern aufweisen.

8. Führungskatheter nach Anspruch 6, bei dem die Wolframcarbidpartikel eine durchschnittliche Größe von kleiner oder gleich 200 Nanometern aufweisen.

9. Führungskatheter nach Anspruch 5, bei dem die Wolframcarbidpartikel 40 bis 80 Gewichtsprozent der distalen Spitze sind bzw. ausmachen.

10. Führungskatheter nach Anspruch 5, bei dem die Wolframcarbidpartikel 70 bis 80 Gewichtsprozent der distalen Spitze ausmachen.

11. Führungskatheter nach Anspruch 1, bei dem die längliche Hülle ferner eine Verstärkungslitze (34) aufweist, die sich entlang einer Länge der Hülle zu einem Punkt proximal der vollständig radioopaken und echogenen distalen Spitze erstreckt.

12. Führungskatheter nach Anspruch 11, bei dem der Punkt proximal der distalen Spitze zwischen 0,25 Inch (6,35 mm) und 0,35 Inch (8,89 mm) von der distalen Spitze beabstandet ist.

13. Führungskatheter nach einem der vorstehenden Ansprüche, bei dem die distale Spitze einen ersten elastischen vorgeformten Bogen beinhaltet.

14. Führungskatheter nach Anspruch 13, bei dem der erste Bogen einen Winkel von größer oder gleich 180° überstreicht.

15. Führungskatheter nach Anspruch 13, bei dem der erste Bogen einen Winkel von 90° überstreicht.

16. Führungskatheter nach Anspruch 13, bei dem der erste Bogen einen Winkel zwischen 10° und 70° überstreicht.

17. Führungskatheter nach einem der Ansprüche 13 bis 16, bei dem die distale Spitze ferner einen zweiten elastischen vorgeformten Bogen aufweist, der sich von dem ersten elastischen vorgeformten Bogen ausgehend erstreckt.

18. Führungskatheter nach Anspruch 17, bei dem die ersten und zweiten Bögen zusammen einen Winkel größer als 90° überstreichen.

19. Führungskatheter nach Anspruch 17, bei dem der erste Bogen einen Winkel zwischen 160° und 180° überstreicht und der zweite Bogen in einer entgegengesetzten Richtung zu dem ersten Bogen einen Winkel zwischen 40° und 80° überstreicht.

20. Führungskatheter nach Anspruch 17, bei dem der erste Bogen und der zweite Bogen eine Form nach Amplatz bilden.

21. Führungskatheter nach einem der Ansprüche 17 bis 20, bei dem die distale Spitze ferner einen dritten elastischen vorgeformten Bogen beinhaltet, der sich von dem zweiten elastischen vorgeformten Bogen ausgehend erstreckt.

22. Führungskatheter nach Anspruch 21, bei dem die ersten, zweiten und dritten Bögen zusammen einen Winkel von mehr als 100° überstreichen.

23. Führungskatheter nach einem der vorstehenden Ansprüche, bei dem die distale Spitze einen sich verjüngenden Übergang aufweist.

24. Führungskatheter nach Anspruch 23, bei dem der sich verjüngende Übergang proximal des ersten elastischen vorgeformten Bogens angeordnet ist.

25. Führungskatheter nach Anspruch 23, wenn dieser von Anspruch 17 abhängt, bei dem der sich verjüngende Übergang proximal des zweiten elastischen vorgeformten Bogens angeordnet ist.

26. Führungskatheter nach Anspruch 23, wenn dieser von Anspruch 21 abhängt, bei dem der sich verjüngende Übergang proximal des dritten elastischen vorgeformten Bogens angeordnet ist.

27. Führungskatheter nach einem der vorstehenden Ansprüche, bei dem das Material, das die distale Spitze bildet, eine Härte zwischen 25 Durometer und 35 Durometer auf einer D-Skala aufweist.

28. Führungskatheter nach Anspruch 1, bei dem
die längliche Hülle ferner ein proximales Segment beinhaltet, wobei das proximale Segment ein distales Ende beinhaltet; und
die vollständig radioopake und echogene distale Spitze mit dem distalen Ende des proximalen Segments über eine Stoßverbindung verbunden ist.

29. Führungskatheter nach Anspruch 28, bei dem die Stoßverbindung mittels Hitze fusioniert bzw. verbunden ist.

30. Führungskatheter nach Anspruch 29, bei dem die Stoßverbindung mittels eines HF-Prozesses gebildet ist.

31. Führungskatheter nach Anspruch 30, bei dem die vollständig radioopake und echogene distale Spitze einen verjüngten Übergang aufweist, der in dem HF-Element bzw. in der HF-Vorrichtung (RF die) ausgebildet ist bzw. wurde.

32. Führungskatheter nach Anspruch 1, bei dem:
die längliche Hülle ferner ein proximales Segment mit einer ersten Steifheit aufweist, das ein distales Ende beinhaltet; und
die vollständig radioopake und echogene distale Spitze mit dem distalen Ende des proximalen Segmentes verbunden ist und eine zweite Steifheit aufweist, welche geringer ist als die erste Steifheit.

33. Führungskatheter nach einem der vorstehenden Ansprüche, bei dem die distale Spitze atraumatisch ist.

34. Führungskatheter nach Anspruch 1, bei dem
der längliche Hülle ferner ein proximales Segment beinhaltet; wobei das proximale Segment ein distales Ende und einen Proximalsegment-Außendurchmesser beinhaltet; und
die vollständig radioopake und echogene distale Spitze mit dem distalen Ende des proximalen Segmentes verbunden ist und einen Distalspitzen-Außendurchmesser beinhaltet, welcher geringer ist als der Proximalsegment-Außendurchmesser.

## Revendications

1. Cathéter-guide comportant une gaine allongée (50) incluant un embout distal entièrement radio-opaque et échogène, l'embout distal (51) ayant une longueur supérieure ou égale à 2,032 mm (0,08 pouce) ; **caractérisé en ce que** ledit cathéter-guide comporte en outre une gaine extérieure (60) recevant la gaine allongée de manière coulissante ; dans lequel la gaine allongée introduite à l'intérieur de la gaine extérieure peut être positionnée de telle sorte que l'embout distal entièrement radio-opaque et échogène fait saillie à partir d'une extrémité distale (63) de la gaine extérieure.

2. Cathéter-guide selon la revendication 1, dans lequel la longueur de l'embout distal est comprise entre 2,032 mm (0,08 pouce) et 5,08 mm (0,2 pouce).

3. Cathéter-guide selon la revendication 1, dans lequel la longueur de l'embout distal est supérieure ou égale à 5,08 mm (0,2 pouce).

4. Cathéter-guide selon la revendication 1, dans lequel la longueur de l'embout distal est comprise entre 10,16 mm (0,4 pouce) et 5,08 cm (2 pouces).

5. Cathéter-guide selon l'une quelconque des revendications précédentes, dans lequel l'embout distal est formé d'un matériau comportant des particules de carbure de tungstène.

6. Cathéter-guide selon la revendication 5, dans lequel les particules de carbure de tungstène ont une taille moyenne inférieure ou égale à 500 nanomètres.

7. Cathéter-guide selon la revendication 6, dans lequel les particules de carbure de tungstène ont une taille moyenne entre 100 nanomètres et 200 nanomètres.

8. Cathéter-guide selon la revendication 6, dans lequel les particules de carbure de tungstène ont une taille moyenne inférieure ou égale à 200 nanomètres.

9. Cathéter-guide selon la revendication 5, dans lequel les particules de carbure de tungstène représentent 40 % à 80 % en poids de l'embout distal.

10. Cathéter-guide selon la revendication 5, dans lequel les particules de carbure de tungstène représentent 70 % à 80 % en poids de l'embout distal.

11. Cathéter-guide selon la revendication 1, dans lequel la gaine allongée inclut en outre une tresse de renfort (34) s'étendant le long d'une longueur de la gaine jusqu'à un point proximal à l'embout distal entièrement radio-opaque et échogène.

12. Cathéter-guide selon la revendication 11, dans lequel le point proximal à l'embout distal est séparé de l'embout distal d'une distance comprise entre 6,35 mm (0,25 pouce) et 8,89 mm (0,35 pouce).

13. Cathéter-guide selon l'une quelconque des revendications précédentes, dans lequel l'embout distal inclut une première courbe préformée élastique.

14. Cathéter-guide selon la revendication 13, dans lequel la première courbe effectue un balayage angulaire supérieur ou égal à 180 °.

15. Cathéter-guide selon la revendication 13, dans lequel la première courbe effectue un balayage angulaire de 90 °.

16. Cathéter-guide selon la revendication 13, dans lequel la première courbe effectue un balayage angulaire entre 10 ° et 70 °.

17. Cathéter-guide selon l'une quelconque des revendications 13 à 16, dans lequel l'embout distal inclut en outre une deuxième courbe préformée élastique s'étendant à partir de la première courbe préformée élastique.

18. Cathéter-guide selon la revendication 17, dans lequel les première et deuxième courbes, associées, effectuent un balayage angulaire supérieur à 90 °.

19. Cathéter-guide selon la revendication 17, dans lequel la première courbe effectue un balayage angulaire entre 160 ° et 180 ° et la deuxième courbe effectue, dans une direction opposée à la première courbe, un balayage angulaire entre 40 ° et 80 °.

20. Cathéter-guide selon la revendication 17, dans lequel la première courbe et la deuxième courbe ont une forme d'Amplatz.

21. Cathéter-guide selon l'une quelconque des revendications 17 à 20, dans lequel l'embout distal inclut en outre une troisième courbe préformée élastique s'étendant à partir de la deuxième courbe préformée élastique.

22. Cathéter-guide selon la revendication 21, dans lequel les première, deuxième et troisième courbes, associées, effectuent un balayage angulaire supérieur à 100 °.

23. Cathéter-guide selon l'une quelconque des revendications précédentes, dans lequel l'embout distal inclut une transition rétrécie.

24. Cathéter-guide selon la revendication 23, dans lequel la transition rétrécie est positionnée proximale à la première courbe préformée élastique.

25. Cathéter-guide selon la revendication 23 lorsqu'elle dépend de la revendication 17, dans lequel la transition rétrécie est positionnée proximale à la deuxième courbe préformée élastique.

26. Cathéter-guide selon la revendication 23 lorsqu'elle dépendant de la revendication 21, dans lequel la transition rétrécie est positionnée proximale à la troisième courbe préformée élastique.

27. Cathéter-guide selon l'une quelconque des revendications précédentes, dans lequel le matériau formant l'embout distal a une dureté entre 25D et 35D lorsque mesurée selon l'échelle D.

28. Cathéter-guide selon la revendication 1, dans lequel la gaine allongée inclut en outre un segment proximal ; le segment proximal incluant une extrémité distale ; et l'embout distal entièrement radio-opaque et échogène est couplé à l'extrémité distale du segment proximal au moyen d'un joint bout à bout.

29. Cathéter-guide selon la revendication 28, dans lequel le joint bout à bout assemblé par thermofusion.

30. Cathéter-guide selon la revendication 29, dans lequel le joint bout à bout est formé par un processus RF.

31. Cathéter-guide selon la revendication 30, dans lequel l'embout distal entièrement radio-opaque et échogène inclut une transition rétrécie formée dans la filière ou le dispositif RF.

32. Cathéter-guide selon la revendication 1, dans lequel :
la gaine allongée inclut en outre un segment proximal ayant une première rigidité et incluant une extrémité distale ; et
l'embout distal entièrement radio-opaque et échogène est couplé à l'extrémité distale du segment proximal et a une seconde rigidité, qui est inférieure à la première rigidité.

33. Cathéter-guide selon l'une quelconque des revendications précédentes, dans lequel l'embout distal est atraumatique.

34. Cathéter-guide selon la revendication 1, dans lequel la gaine allongée inclut en outre un segment proximal ; le segment proximal incluant une extrémité distale et un diamètre extérieur de segment proximal ; et l'embout distal entièrement radio-opaque et échogène est couplé à l'extrémité distale du segment proximal et inclut un diamètre extérieur d'embout distal, qui est inférieur au diamètre extérieur de segment proximal.
